# EUROPEAN PATENT SPECIFICATION

(11) **EP 2 458 338 B1**
(45) Date of publication and mention of the grant of the patent: **31.12.2014**
(21) Application number: 10192636.8
(22) Date of filing: 25.11.2010
(51) Int. Cl.: G01C 22/00

(54) **Pedometer With Shoe Mounted Sensor And Transmitter**
Schrittzähler mit am Schuh montierten Sensor und Sender
Pédomètre doté d'un capteur et d'un transmetteur montés sur la chaussure

(43) Date of publication of application: 30.05.2012
(73) Proprietor: Silicon Valley Micro E Corporation, San Jose CA 95119 (US)
(72) Inventor: Zhu, Shengbo, San Jose, California 95123 (US); Huang, Su Shiong, Bellevue, Washington 98004 (US)
(74) Representative: Boult Wade Tennant

(56) References cited:
- WO-A1-98/00683
- WO-A1-99/65385
- WO-A1-2007/008352
- WO-A1-2008/042720
- DE-U1-202006 009 810

## Description

### BACKGROUND OF THE INVENTION

This invention relates to pedometers used to measure pedestrian step counts and calculate distances traveled on foot. More particularly, this invention relates to a pedometer that comprises a shoe mounted system that acquires pedestrian performance data and transmits calculated results to a separate display unit.

Pedometers are being increasingly used by both professional and amateur fitness enthusiasts as an aid in monitoring and evaluating exercise routines. By using a pedometer, a person can measure and record a variety of data parameters, such as: step count, distance traveled, speed, and calories burned to name a few. These parameters are useful in determining the effectiveness and efficiency of a particular fitness program. Additionally, a pedometer may be used as a motivational device by providing a person with a way to track their daily physical activity level and correspondingly establish increased activity level targets. The use of a pedometer, in many instances, has motivated people to significantly increase their physical activity levels resulting in lower blood pressure, weight loss and better overall fitness.

Several different types of pedometers are known and are currently available. These known pedometers utilize a range of technologies to determine step counts and distances. One classic type of known pedometer is a mechanical device that uses a pendulum to detect physical motion and then convert that motion into a step count. A person typically wears the mechanical pedometer on their belt in a substantially vertical orientation. As the person walks, their hips induce a swinging motion into the pedometer, which in turn causes a weighted pendulum to move within the pedometer housing. The inertia of the pendulum is sensed by means of a ratchet mechanism or mechanical stop, which thereby advances a mechanical counter. While somewhat useful, pedometers using a pendulum to detect steps frequently record "false steps" or erroneous movements such as bending over and leaning. Moreover, pendulum actuated pedometers are sensitive to proper vertical alignment and usually require mechanical adjustment in relation to the gait/stride of the user in order to accurately record steps and convert the number of steps to a distance value.

Other types of known pedometers use electro-mechanical systems to detect and record a step count. Once such pedometer counts steps by means of one or more electro-mechanical switches embedded within a shoe. As a person steps, the switch is either opened or closed creating an electrical signal which is used to increment an electronic counter. Although this type of pedometer is usually more accurate than pendulum-type pedometers, false steps are still frequently recorded such as when a person shifts their weight from one foot to another. In addition, it is more than a trivial task to incorporate the switches in a shoe so that the switches reliably sense each step. Further, the switches are prone to contamination *in situ* and are susceptible to wear given the harsh environment in which they are located.

More sophisticated electro-mechanical pedometers use one or more accelerometers and microprocessors properly programmed to detect pedestrian steps. These pedometers generally have 1-, 2- or 3-axis accelerometers to measure accelerations and generate electronic signals corresponding to physical movement. The software in the microprocessor then processes the electronic acceleration signals to determine step count, step frequency and stride length. While this type of pedometer is useful and possibly more accurate than pendulum based and switch based pedometers at high-frequency step counts, false steps and erroneous distances can be generated during low speed movement. Additionally, improper axial alignment of the accelerometers during use can adversely affect the accuracy of these pedometers.

In one known accelerometer-type pedometer described in U.S. Patent Number 6,145,389 to Ebeling et al., November 14, 2000, an accelerometer is attached to a shoe and a microprocessor uses the signals generated by the accelerometer to calculate stride length. This pedometer requires that the accelerometer be carefully aligned such that the axis of acceleration measurement is substantially aligned with the direction of pedestrian foot travel. Correspondingly, should improper axial alignment of the accelerometer occur during use, incomplete and inaccurate measurements can result.

In another known accelerometer-type pedometer described in U.S. Patent Number 6,175,608 to Pyles et al., January 16, 2001, an inertial device is mounted to the waist, chest, or leg of a user to determine stride count. The inertial device of this pedometer detects gross physical movements similar to pendulum-type pedometers. While this type of pedometer is useful, false steps or irrelevant movements, such as bending over and leaning, may be erroneously recorded as steps. Moreover, since the inertial device determines step count based on acceleration, low-speed steps may not be accurately detected. Additionally, improper alignment of the inertial device during use may adversely affect the accuracy of these pedometers.

Efforts to provide a pedometer devoid of the above-noted disadvantages have not met with success to date.

WO 99/65385 discloses a pedometer assembly in which a magnet is strapped to one shoe of a pair and a Hall effect sensor is strapped to the other shoe. The Hall effect sensor provides a signal indicating the strength of the magnetic field generated by the magnet which corresponds to the distance separating the magnet and the sensor. The relative speed of movement is determined from the rate of change of the amplified output voltage of the Hall effect sensor.

WO 98/00683 discloses a similar pedometer assembly with a signal generator associated with one foot and a receiver associated with the other foot. A signal generator comprises infrared diodes and the receiver comprises infrared sensors.

DE 20 2006 009 810 discloses a pedometer in which a permanent magnet in a first shoe is used to power two separate RFID circuits longitudinally spaced in a second shoe.

### SUMMARY OF THE INVENTION

Examples of the invention comprise a pedometer which is devoid of the above-noted disadvantages, which substantially reduces false step count readings and provides high accuracy at low speeds, and which is relatively simple to implement in existing foot wear.

The invention comprises a pedometer as set out in claim 1. The first and second signal generators and the sensor are preferably aligned on the first and second shoes in facing relation when the first and second shoes are worn by a user so as to maximize the incidence of detection of the signals from the first and second signal generators by the sensor.

Preferably, the first and second signal generators are mounted adjacent the inner margin of the first shoe, and the sensor is mounted adjacent the inner margin of the second shoe.

The first and second signal generators and the sensor are alternately implemented using a variety of technologies. In a magnetic technology implementation, the first and second signal generators comprise permanent magnets; and the sensor comprises a device such as a Hall effect sensor or an MR sensor for converting the magnetic fields generated by the permanent magnets to corresponding electrical signals. In an optical technology implementation, the first and second signal generators comprise light radiation sources, such as light emitting diodes; and the sensor comprises a device for converting the light radiation generated by the light radiation sources to corresponding electrical signals. In an r.f. technology implementation, the first and second signal generators comprise RFID tags for generating r.f. signals of known frequency; and the sensor comprises an RFID reader device for converting r.f. signals received from the RFID tags to corresponding electrical signals. The RFID signal generator tags may comprise either active or passive RFID tags.

The pedometer may further include a transmitter coupled to the microcontroller unit for transmitting the pedestrian performance data to a receiver/ display unit to provide real time user feedback.

Pedometers fabricated according to the teachings of the invention are simple to incorporate into foot wear at the point of manufacture or as an after market item at relatively low cost. Such pedometers are capable of providing accurate pedestrian performance data, such as foot speed, step count, distance travelled, cadence and many other performance parameters of potential interest to users.

For a fuller understanding of the nature and advantages of examples of the invention, reference should be made to the ensuing detailed description taken in conjunction with the accompanying drawings.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention. In the following description, various embodiments of the present invention are described with reference to the following drawings, in which:
FIG. 1 is schematic view of a bipedal walk cycle;
FIG. 2 is an elevational view illustrating a shoe mounted pedometer embodying the invention;
FIG. 3 is a block diagram of a pedometer having a shoe mounted signal generator, a sensor and transmitter and an associated separate pedestrian parameter display unit;
FIG. 4 is an enlarged schematic view of a typical biped walk cycle;
FIG. 5 is a graph illustrating sensor signals versus time; and
FIG. 6 is a graph illustrating foot speed versus time.

### DETAILED DESCRIPTION OF THE PREFERRED EMBODIMENTS

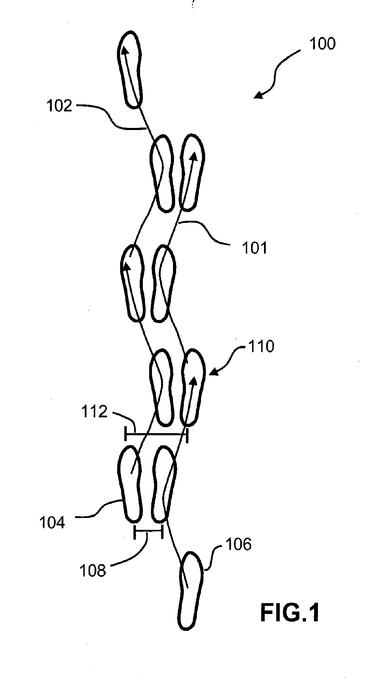

Turning now to the drawings, Fig. 1 is a schematic view of a typical bipedal walk cycle 100. As seen in this Fig., a typical human or bipedal walk cycle 100 for generally linear forward motion incorporates a right foot sinuous path 101 and a left foot sinuous path 102. When both the right and left foot sinuous paths 101, 102 are combined, a biped walk cycle 100 producing forward generally linear motion is achieved. To begin a walk cycle 100, a forward foot 104 carries the majority of the weight of a person. The rearward foot 106 is then lifted and moved in a generally forward direction, as indicated by the directional arrows. As the rearward foot 106 moves towards the forward foot 104, the motion of rearward foot 106 curves inwards towards the forward foot 104 to form an arcuate path. A minimum interspatial distance 108 between the two feet 104, 106 is defined when the initially forward foot 104 and the initially rearward foot 106 achieve a minimum separation distance. At this point in the walk cycle 100, the initially rearward foot 106 usually carries no body weight and the initially forward foot 104 bears the entire body weight of the person. Next, the initially rearward foot 106 curves away from the initially forward foot 104 and contacts the ground surface at a position 110 that is located generally forward of the initially forward foot 104 and achieves a maximum interspatial distance 112. As initially rearward foot 106 makes supportive contact with the ground surface, the weight of the person is distributed between both initially forward and rearward feet 104, 106.

As shown in Fig. 1, the walk cycle 100 is repeated for the initially forward foot 104 in a similar manner as previously described but with the roles of the feet reversed. Thus, with foot 106 carrying the majority of the weight of the person, foot 104 is then lifted and moved in a generally forward direction, as indicated by the directional arrows. As foot 104 moves towards foot 106, the path of foot 104 curves inwards towards foot 106 to form an arcuate path. The minimum interspatial distance 108 between the two feet 104, 106 is defined when foot 104 and foot 106 achieve a minimum separation distance. At this point in the walk cycle 100, foot 104 usually carries no body weight and foot 106 bears the entire body weight of the person. Next, foot 104 curves away from foot 106 and contacts the ground surface at a position that is located generally forward of foot 106 and achieves the maximum interspatial distance 112. As foot 104 makes supportive contact with the ground surface, the weight of the person is distributed between both feet 104, 106.

The exact form of right and left sinuous paths 101, 102 is generally a product of body mechanics, such as hip rotation, weight transfer and numerous other postural alignments that are required for bipedal motion. The minimum interspatial distance 108 can be less than 1.27cm (0.5 inch) and is generally dependant upon the physical structure and other attributes of the person walking. The maximum interspatial distance 112 is generally about shoulder width of the person but may vary depending on the gait and stride of the person.

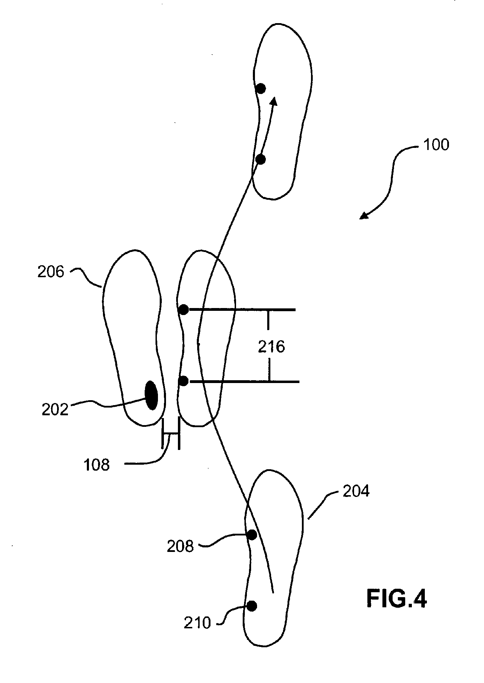

Due to the close proximity of both feet at the minimum interspatial distance 108, it is possible to implement shoe mounted signal generators and a proximity sensor to detect when the feet pass each other during the walk cycle. Detection of the passing feet is discussed in further detail below with respect to Fig. 4.

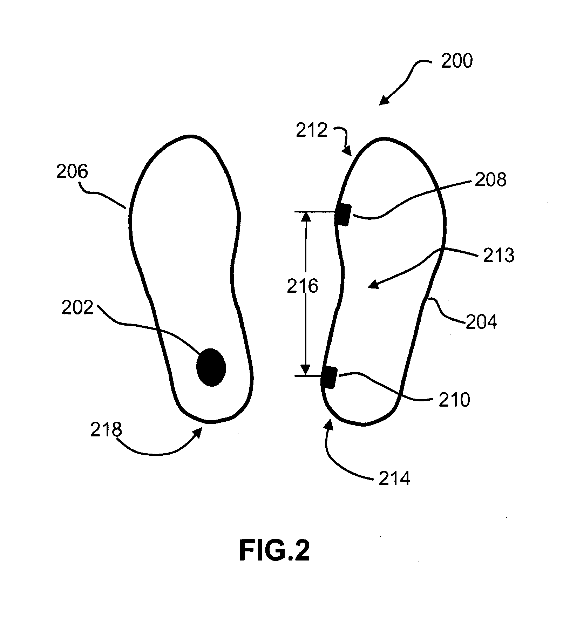

Reference is now made to Fig. 2, which is an elevational view illustrating a pedometer generally designated with reference numeral 200 comprising a shoe mounted system capable of acquiring real time pedestrian performance data during ambulatory pedestrian motion. As shown in Fig. 2, pedometer 200 is incorporated into a right shoe 204 and a corresponding left shoe 206. Both right and left shoes 204, 206 generally form a matching pair of shoes suitable for wear and use in ambulatory motion such as walking, running, and jogging. The right shoe 204 has mounted thereon a first signal generator 208 and a second signal generator 210. The first signal generator 208 is positioned adjacent a relatively forward portion 212 of right shoe 204 and the second signal generator 210 is positioned adjacent a relatively rearward portion 214 of right shoe 204. Both first and second signal generators 208, 210 are preferably positioned along the inner margin of right shoe 204 so as to be nearest to the corresponding left shoe 206 and proximate an instep region 213 of right shoe 204. In an exemplary embodiment, first and second signal generators 208, 210 are each fabricated from permanent magnetic material that produces magnetic fields sufficient to reach a region of left shoe 206 at which a sensor 202 is located. As will be apparent to those of ordinary skill in the art, many different types of magnetic material may be used, such as ferromagnetic materials (e.g., cobalt and nickel) and ferrimagnetic materials, and composites such as Alnico, Ticonal, and sintered composites of powdered iron oxide and barium/strontium carbonate ceramic. A signal generator longitudinal separation distance 216 defines a fixed distance between first and second signal generators 208, 210 along the generally longitudinal axis of right shoe 204. In an exemplary embodiment, longitudinal separation distance 216 is approximately 12.7cm (5 inches); however, it is contemplated that other fixed dimensions for longitudinal separation distance 216 may be used depending on the relative size and configuration of the shoes. In an exemplary embodiment, signal generators 208, 210 are embedded within a sole (not shown) forming a part of right shoe 204. It is understood that signal generators 208, 210 may be incorporated into other components of right shoe 204 by molding or adhesing, or mechanically attached to an appropriate portion of right shoe 204 by any suitable attachment technique, such as loop-and-hook fastener material sold under the trademark Velcro.

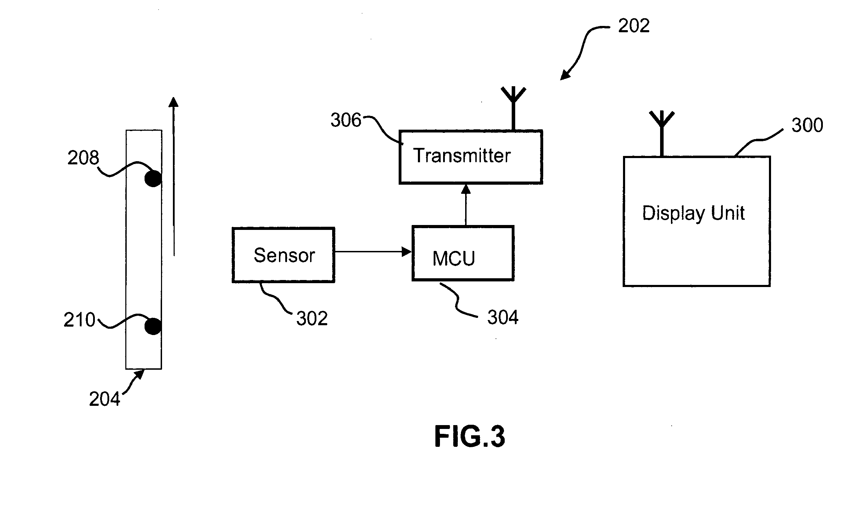

Left shoe 206 has mounted thereon a sensor and transmitter assembly 202 located in a fixed position of left shoe 206. In an exemplary embodiment, sensor and transmitter assembly 202 comprises at least one proximity sensor (such as a Hall effect sensor) capable of sensing the magnetic field signals generated by generators 208, 210 mounted on right shoe 204, a microcontroller unit and a transmitter. These elements are discussed in further detail below with respect to Fig. 3. It is contemplated that sensor and transmitter assembly 202 is embedded within a sole (not shown) forming a part of left shoe 206. In alternate embodiments, sensor and transmitter assembly 202 may be coupled to other components and regions of left shoe 204. Although signal generators 208, 210 have been described with respect to the right shoe and the sensor and transmitter assembly 202 has been described with respect to the left shoe 206, one having ordinary skill in the art will readily appreciate that reversing the right and left shoe configurations is also possible.

Fig. 3 is a block diagram of the pedometer of Fig. 2 having shoe mounted signal generators 208, 210, and a shoe mounted sensor and transmitter assembly 202 and an associated separate display unit 300. As seen in this Fig., a proximity sensor 302 is positioned in operable range with first and second signal generators 208, 210 such that magnetic impulse signals are induced in sensor 302 when relative motion exists between right and left shoes 204, 206 in such a manner that signal generators 208, 210 pass by the region of left shoe 206 within the operational range of proximity sensor 302. In the magnetic implementation being described, proximity sensor 302 is preferably a Hall-effect sensor available from Allegro Microsystems, Inc., part number: A1395SEHLT. Alternatively, proximity sensor 302 may be an MR sensor available from Honeywell Microelectronics, part number: HMC1001.

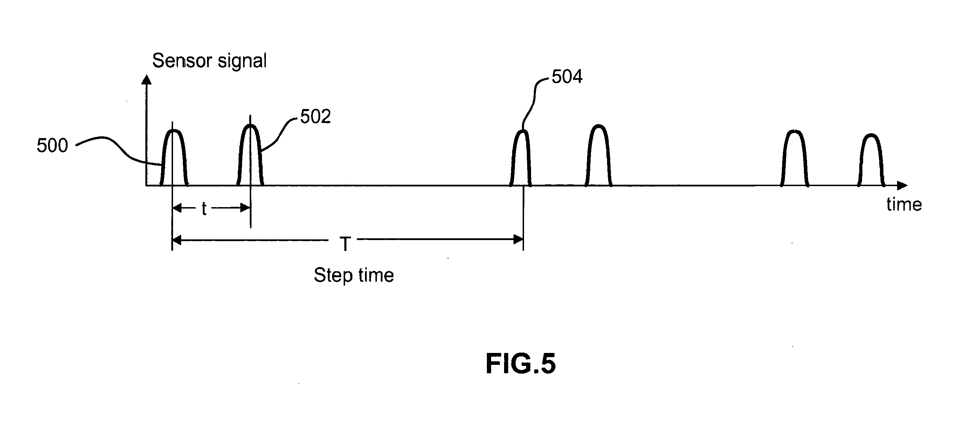

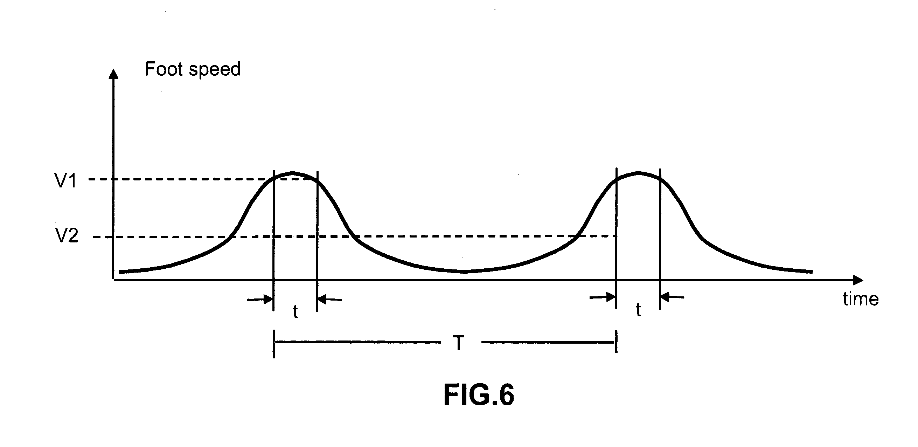

Proximity sensor 302 is operatively coupled with a microcontroller unit (MCU) 304 such that magnetic impulse signals received by proximity sensor 302 are converted to electrical signals which are coupled to MCU 304 for various signal processing functions (discussed in further detail below with respect to Figs. 5 & 6). MCU 304 is operatively coupled with transmitter 306 for wireless transmission of processed pedestrian performance data to display unit 300. Performance data may include: total steps, steps per minute, instantaneous foot speed, average foot speed, cadence, total distance travelled, distance per stride, calories burned and other parametric data produced by MCU 304. MCU 304 and transmitter 306 are preferably combined in a type AT3 chipset available as SensRcore part number nRF24LO1 from ANT of Cochrane, Alberta, Canada. Transmitter 306 communicates wirelessly with display unit 300 by either unidirectionally or bidirectionally transferring performance data and step information thereto. It is contemplated that display unit 300 may be a third party performance monitoring device or fitness computer such as the Edge 705 unit available from Garmin Ltd., part number 010-00555-20. In an alternate embodiment, transmitter 306 may be configured to communicate and transfer performance data to other electronic devices such as a cell phone, an Mp3 player or other portable display device.

In an exemplary embodiment, the wireless communication protocol used between transmitter 306 and display unit 300 is a wireless sensor network communication protocol commonly referred to as "ANT" available from Dynastream Innovations, Inc. of Cochrane, Alberta, Canada. Some features of the ANT protocol include low power consumption, low cost overhead, and the ability of multiple transceivers to co-exist in close proximity to other similar transceivers. The ANT protocol has an estimated efficiency of about 47 percent due to various programming configurations that reduce power consumption in a standby state. However, one having ordinary skill in the art would readily appreciate that other types of wireless communication protocols such as Bluetooth or ZigBee (based upon IEEE standard 802.15.4) may be utilized to facilitate data transfer between transmitter 306 and display unit 300.

A suitable source of D.C. electrical power, such as a battery (not shown) is used to power the system elements 302, 304, and 306 shown in Fig. 3. In the alternative, the magnetic fields generated by magnets 208, 210 can serve as an energy source when combined with a coil and a D.C. rectifier circuit included in assembly 202. This arrangement eliminates the need to replace a battery when the useful energy is depleted from the battery. Display unit 300 is provided with a separate power source, such as a battery.

Reference is now made to Figs. 4 and 5, in which Fig. 4 is an enlarged schematic view of a representative portion of a bipedal ambulatory cycle 100 and Fig. 5 is a graph illustrating sensor signals versus time. The pedometer of the present invention generates a step count and step time as one shoe passes by the other shoe. For example, in Fig. 4, as right shoe 204 follows the right foot sinuous path 101, first and second signal generators 208, 210 are successively brought into close proximity with sensor and transmitter assembly 202. At the minimum interspatial distance 108, and as the first signal generator 208 passes by the proximity sensor within assembly 202, a first impulse signal 500 (Fig. 5) is generated by sensor 302 (Fig. 3) with the maximum value occurring at the point of closest approach between generator 208 and sensor 302. This first impulse signal is coupled to MCU304 (Fig. 3) in sensor and transmitter assembly 202. As the right shoe 204 moves an additional distance forward, equal to the fixed longitudinal separation distance 216, the second signal generator 210 passes the proximity sensor within assembly 202 and generates a second impulse signal 502 that is received by MCU 304. This pair of first and second impulse signals 500, 502 is separated by a first time interval "t", which can be determined by MCU 304 given the known separation distance between signal generators 208, 210. As the left shoe 206 progresses to the next step in the walk cycle 100, a third impulse signal 504 is generated at a second time interval as the proximity sensor 302 contained within sensor and transmitter assembly 202 moves by the second signal generator 210. Once the third impulse signal 504 is generated, the value of a parameter termed step time "T" can be determined by MCU 304.

Upon acquiring the real time pedestrian data "t" and "T" as discussed above, various other performance parameters can be calculated such as cadence, speed and total distance traveled. Pedometer cadence is calculated by dividing the total number of steps by the sum total of step time. The cadence value may then be converted into various units such as steps/minute by applying standard time conversions. For total distance traveled, and referring to Fig. 6, first an average foot speed V1 is determined over first time interval "t". Average foot speed is calculated by using the fixed separation distance between the first and second signal generators 208, 210 and first time interval "t". In an exemplary embodiment, the longitudinal distance separating the first and second signal generators 208, 210 is 12.7cm (5 inches) and the resulting foot speed over first time interval "t" is defined by V1=12.7/t cms per second (5/t inches per second). Second, a coefficient "K" is used to proportionally scale average foot speed V1 to average step speed V2. Coefficient K may be determined either by calibration based on actual user stride length or by assembling a table of average stride lengths based upon standard physical attributes relating stride length to the height of a person. Once "K" is determined, the equation for step speed is defined by V2=KV1. Third, step length "d" is determined by multiplying V2 by "T", d=V2T. The total number of steps "N" is determined by accumulating the total number of step times "T". Finally, to obtain total distance traveled "D", step length "d" is multiplied by the total number of steps "N", D=Nd. All the above algorithms can be readily implemented in MCU 204 using standard techniques.

The resulting performance data determined by MCU 304 can be stored in MCU 304 memory for subsequent analysis, and also transmitted by transmitter 306 to display unit 300 to provide real time performance data feedback to the user.

Although described above as operating in the magnetic domain, signal generators 208, 210 and sensor 302 may be implemented using other technologies, such as optical and r.f. technologies. For example, for an implementation using optical technology signal generators 208, 210 may comprise light emitting diodes (LEDs) which generate light beams of a known wave length, and sensor 302 may comprise an optical sensor for sensing light radiation at the LED wave length. In such an implementation, a source of electrical energy, such as a battery, must be provided to power the LED signal generators 208, 210. Similarly, for an implementation using r.f. technology signal generators 208, 210 may comprise RFID tags which generate r.f. signals at a known frequency and sensor 302 may comprise an RFID reader/interrogator unit capable of sensing r.f. signals at the known frequency. The RFID tags may comprise active or passive RFID tags. If active RFID tags are employed, a source of electrical energy, such as a battery, must be provided to power the RFID tags. If passive RFID tags are employed, they will be powered by the r.f. interrogation signals from sensor 302 and no separate electrical power source is required for the signal generators 208, 210. One suitable choice for a passive RFID tag is an Atmel type ATA5577 RFID tag available from Atmel Corporation of San Jose, California. One suitable choice for an RFID reader/interrogator is an Atmel type ATA5577 device, also available from Atmel Corporation of San Jose, California.

As will now be apparent, pedometers fabricated according to the teachings of the present invention offer accuracy and convenience advantages over known pedometers. Firstly, the use of a shoe mounted proximity sensor and signal generator provides improved accuracy for determining step count. This improved accuracy results from generating impulse signals each time the feet pass by each other. In addition, pedometers fabricated according to the teachings of the present invention reduce the number of recorded "false steps" by eliminating reliance on mechanical movements and axial alignment of accelerometers. Further, by employing a shoe-mounted sensor and transmitter that wirelessly communicates with a separate display, greater user convenience is achieved over pedometers that have integral display units. Lastly, by employing the proximity sensor and signal generator configuration shown in Figs. 2-5, a high level of step count accuracy can be achieved at very low walking speeds.

While the invention has been described with reference to particular embodiments, various modifications, alternate constructions and equivalents may be employed. For example, while certain circuit components have been disclosed, other equivalent units may be employed, as desired. Therefore, the above should not be construed as limiting the invention, which is defined by the appended claims.

## Claims

1. A pedometer comprising:
a pair of shoes (204, 206);
a first signal generator (208) carried by a first portion of a first shoe (204) of said pair of shoes; **characterised in that** the pedometer also comprises
a second signal generator (210) carried by a second portion of said first shoe (204), said first and second signal generators (208, 210) being separated by a fixed distance (216) extending longitudinally of said first shoe (204);
a sensor assembly (202) coupled with a second shoe (206) of said pair, said sensor assembly (202) including a proximity sensor (302) mounted on said second shoe (206) for sensing signals generated by said first and second signal generators (208, 210) and for generating corresponding electrical signals, and a microcontroller unit (304) having an input coupled to said proximity sensor (302) for receiving said corresponding electrical signals and converting said corresponding electrical signals into pedestrian performance data.

2. The pedometer of claim 1 wherein said first and second signal generators (208, 210) and said proximity sensor (302) are aligned on said first and second shoes (204, 206) in facing relation when said first and second shoes are worn by a user.

3. The pedometer of claim 1 or claim 2 wherein said first and second signal generators (208, 210) are mounted adjacent the inner margin of said first shoe (204).

4. The pedometer of claim 3 wherein said proximity sensor (302) is mounted adjacent the inner margin of said second shoe (206).

5. The pedometer of any preceding claim wherein said first and second signal generators (208, 210) comprise permanent magnets; and wherein said proximity sensor (302) comprises a device for converting the magnetic fields generated by said permanent magnets to corresponding electrical signals.

6. The pedometer of claim 5 wherein said proximity sensor (302) comprises a Hall effect sensor device.

7. The pedometer of claim 5 wherein said proximity sensor (302) comprises an MR sensor device.

8. The pedometer of any of claims 1 to 4 wherein said first and second signal generators (208, 210) comprise light radiation sources; and wherein said proximity sensor (302) comprises a device for converting the light radiation generated by said light radiation sources to corresponding electrical signals.

9. The pedometer of claim 8 wherein said light radiation sources are light emitting diodes.

10. The pedometer of any of claims 1 to 4 wherein said first and second signal generators (208, 210) comprise RFID tags for generating r.f. signals of known frequency; and wherein said proximity sensor (302) comprises an RFID reader device for converting r.f. signals received from said RFID tags to corresponding electrical signals.

11. The pedometer of claim 10 wherein said RFID tags are active RFID devices.

12. The pedometer of claim 10 wherein said RFI D tags are passive RFI D devices.

13. The pedometer of any preceding claim further including a transmitter (306) coupled to said microcontroller unit (304) for transmitting said pedestrian performance data to a receiver/ display unit (300) to provide real time user feedback.

14. The pedometer of any preceding claim wherein said first and second signal generators (208, 210) are located along the inner margin of said first shoe (204) in the region of the instep of a foot inserted into said first shoe (204).

## Patentansprüche

1. Pedometer, das aufweist:
ein Paar Schuhe (204, 206),
einen ersten Signalgenerator (208), der von einem ersten Teil eines ersten Schuhs (204) des Schuhpaares getragen wird,
**dadurch gekennzeichnet, dass** das Pedometer ferner aufweist:
einen zweiten Signalgenerator (210), der von einem zweiten Teil des ersten Schuhs (204) getragen wird, wobei der Abstand zwischen dem ersten und dem zweiten Signalgenerator (208, 210) durch eine feststehende Distanz (216) gegeben ist, die sich längs des ersten Schuhs (204) erstreckt,
eine Sensorbaugruppe (202), die mit dem zweiten Schuh des Schuhpaares verbunden ist, wobei die Sensorbaugruppe (202) einen Näherungssensor (302) aufweist, der an dem zweiten Schuh (206) befestigt ist, um Signale wahrzunehmen, die von dem ersten und dem zweiten Signalgenerator (208, 210) erzeugt werden, und um entsprechende elektrische Signale zu erzeugen,
sowie eine Microcontrollereinheit (304), die einen mit dem Näherungssensor (302) verbundenen Eingang zum Erhalt der entsprechenden elektrischen Signale und zum Konvertieren der entsprechenden elektrischen Signale in Fußgänger-Leistungsdaten aufweist.

2. Pedometer nach Anspruch 1, worin der erste und der zweite Signalgenerator (208, 210) sowie der Näherungssensor (302) an dem ersten bzw. dem zweiten Schuh (204, 206) so ausgerichtet sind, dass sie, wenn ein Benutzer den ersten und zweiten Schuh trägt, einander zugewandt sind.

3. Pedometer nach Anspruch 1 oder Anspruch 2, worin der erste und der zweite Signalgenerator (208, 210) an den inneren Rand des ersten Schuhs (204) angrenzend angebracht sind.

4. Pedometer nach Anspruch 3, worin der Näherungssensor (302) an den inneren Rand des zweiten Schuhs (206) angrenzend angebracht ist.

5. Pedometer nach einem der vorhergehenden Ansprüche, worin der erste und der zweite Signalgenerator (208, 210) Dauermagnete aufweisen und worin der Näherungssensor (302) eine Vorrichtung zum Umwandeln der von den Dauermagneten erzeugten magnetischen Felder in elektrische Signale aufweist.

6. Pedometer nach Anspruch 5, worin der Näherungssensor (302) eine Halleffekt-Sensoreinrichtung umfasst.

7. Pedometer nach Anspruch 5, worin der Näherungssensor (302) eine MR-Sensoreinrichtung umfasst.

8. Pedometer nach einem der Ansprüche 1 bis 4, worin der erste und der zweite Signalgenerator (208, 210) Lichtstrahlungsquellen aufweisen und worin der Näherungssensor (302) eine Einrichtung zum Umwandeln der von den Lichtstrahlungsquellen erzeugten Lichtstrahlung in entsprechende elektrische Signale aufweist.

9. Pedometer nach Anspruch 8, worin Licht emittierende Dioden die Lichtstrahlungsquellen bilden.

10. Pedometer nach einem der Ansprüche 1 bis 4, worin der erste und der zweite Signalgenerator (208, 210) RFID-Transponder zum Erzeugen von Funksignalen bekannter Frequenz aufweisen, und worin der Näherungssensor (302) eine RFID-Leseeinrichtung zum Umwandeln von Funksignalen, die von den RFID-Transpondern empfangen werden, in entsprechende elektrische Signale aufweist.

11. Pedometer nach Anspruch 10, worin es sich bei den RFID-Transpondern um aktive RFID-Vorrichtungen handelt.

12. Pedometer nach Anspruch 10, worin es sich bei den RFID-Transpondern um passive RFID-Vorrichtungen handelt.

13. Pedometer nach einem der vorhergehenden Ansprüche, das ferner einen mit der Microcontrollereinheit (304) verbundenen Sender (306) zum Senden der Fußgänger-Leistungsdaten an eine Empfänger/Anzeige-Einheit (300) aufweist, um einen Benutzer mit einer Echtzeit-Rückmeldung zu versorgen.

14. Pedometer nach einem der vorhergehenden Ansprüche, worin der erste und der zweite Signalgenerator (208, 210) entlang des inneren Rands des ersten Schuhs (204) im Bereich des Spanns eines in den ersten Schuh (204) eingeführten Fußes angeordnet sind.

## Revendications

1. Pédomètre comprenant :
une paire de chaussures (204, 206) ;
un premier générateur de signaux (208) supporté par une première portion d'une première chaussure (204) de ladite paire de chaussures ;
**caractérisé en ce que** le pédomètre comprend également
un deuxième générateur de signaux (210) supporté par une seconde portion de ladite première chaussure (204), lesdits premier et deuxième générateurs de signaux (208, 210) étant séparés d'une distance fixe (216) s'étendant longitudinalement de ladite première chaussure (204) ;
un ensemble de capteur (202) couplé à une seconde chaussure (206) de ladite paire, ledit ensemble de capteur (202) incluant un capteur de proximité (302) monté sur ladite seconde chaussure (206) pour détecter les signaux produits par lesdits premier et deuxième générateurs de signaux (208, 210) et pour produire des signaux électriques correspondants, et une unité de microcontrôleur (304) ayant une entrée couplée audit capteur de proximité (302) pour recevoir lesdits signaux électriques correspondants et pour convertir lesdits signaux électriques correspondants en données de performance pédestres.

2. Pédomètre selon la revendication 1, dans lequel lesdits premier et deuxième générateurs de signaux (208, 210) et ledit capteur de proximité (302) sont alignés sur lesdites première et seconde chaussures (204, 206) selon une relation se faisant face l'une à l'autre lorsque lesdites première et seconde chaussures sont portées par un utilisateur.

3. Pédomètre selon la revendication 1 ou la revendication 2, dans lequel lesdits premier et deuxième générateurs de signaux (208, 210) sont montés d'une manière adjacente à la marge intérieure de ladite première chaussure (204).

4. Pédomètre selon la revendication 3, dans lequel ledit capteur de proximité (302) est monté d'une manière adjacente à la marge intérieure de ladite seconde chaussure (206).

5. Pédomètre selon l'une quelconque des revendications précédentes, dans lequel lesdits premier et deuxième générateurs de signaux (208, 210) comprennent des aimants permanents ; et où ledit capteur de proximité (302) comprend un dispositif pour convertir les champs magnétiques produits par lesdits aimants permanents en des signaux électriques correspondants.

6. Pédomètre selon la revendication 5, dans lequel ledit capteur de proximité (302) comprend un dispositif de détection à effet Hall.

7. Pédomètre selon la revendication 5, dans lequel ledit capteur de proximité (302) comprend un dispositif de détection MR.

8. Pédomètre selon l'une quelconque des revendications 1 à 4, dans lequel lesdits premier et deuxième générateurs de signaux (208, 210) comprennent des sources de rayonnement de lumière ; et où ledit capteur de proximité (302) comprend un dispositif pour convertir le rayonnement de lumière produit par lesdites sources de rayonnement de lumière en des signaux électriques correspondants.

9. Podomètre selon la revendication 8, dans lequel lesdites sources de rayonnement de lumière sont des diodes émettrices de lumière.

10. Pédomètre selon l'une quelconque des revendications 1 à 4, dans lequel lesdits premier et deuxième générateurs de signaux (208, 210) comprennent des étiquettes RFID pour produire des signaux r.f. d'une fréquence connue ; et où ledit capteur de proximité (302) comprend un dispositif de lecture RFID pour convertir les signaux r.f. reçus desdites étiquettes RFID en des signaux électriques correspondants.

11. Pédomètre selon la revendication 10, dans lequel lesdites étiquettes RFID sont des dispositifs RFID actifs.

12. Pédomètre selon la revendication 10, dans lequel lesdites étiquettes RFID sont des dispositifs RFID passifs.

13. Pédomètre selon l'une quelconque des revendications précédentes, incluant en outre un transmetteur (306) couplé à ladite unité de microcontrôleur (304) pour transmettre lesdites données de performance pédestre à une unité de réception/affichage (300) pour réaliser un retour d'information utilisateur en temps réel.

14. Pédomètre selon l'une quelconque des revendications précédentes, dans lequel lesdits premier et deuxième générateurs de signaux (208, 210) sont situés le long de la marge intérieure de ladite première chaussure (204) dans la région du cou-de-pied d'un pied inséré dans ladite première chaussure (204).
